# EUROPEAN PATENT SPECIFICATION

(11) **EP 1 412 009 B1**
(45) Date of publication and mention of the grant of the patent: **13.02.2008**
(21) Application number: 02769383.7
(22) Date of filing: 06.05.2002
(51) Int. Cl.: A61M 16/00, A62B 9/02

(54) **VALVED AEROSOL TEE ADAPTER ASSEMBLY**
AEROSOL-T-ADAPTER-ANORDNUNG MIT VENTIL
ENSEMBLE ADAPTATEUR AEROSOL A VALVE EN T

(30) Priority: 07.05.2001 US 850870
(43) Date of publication of application: 28.04.2004
(73) Proprietor: Hudson Respiratory Care Inc., Temecula, CA 92589-9020 (US)
(72) Inventor: LOESCHER, Thomas, C., Rancho Santa Fe, CA 92067 (US)
(74) Representative: Mackenzie, Andrew Bryan
(86) International application number: PCT/US2002/014561
(87) International publication number: WO 2002/089887

(56) References cited:
- US-A- 4 506 665
- US-A- 4 510 933
- US-A- 4 951 661
- US-A- 6 135 108

## Description

### Background of the Invention

In respiratory therapy, treatment with an aerosol directed to the patent via a ventilator circuit is often prescribed. For such practice, a nebulizer is attached to a side port of a Tee connector in the ventilator circuit. Ventilation is temporarily interrupted while the Tee connector and nebulizer are attached to and removed from the circuit. Moreover, opening the respiratory circuit to atmosphere results in exposure of other patients and attending personnel to bacteria, fluids, etc. from the patient present in the circuit. During the aerosol treatment, fluid in the Tee connector from condensation or from the nebulized aerosol particles may drain back into the nebulizer, thereby contaminating the medicament fluid to be nebulized.

A quick-connect adapter valve for connecting a nebulizer to a respiratory circuit is disclosed in U.S. Patent 4,951,661, which shows the features of the preamble of claim 1. Although the apparatus allows the respiratory circuit to remain closed and without interrupting ventilation when connecting a nebulizer for aerosol treatment, the device has some disadvantageous structural components and features. The present invention is directed to an apparatus which provides for securing a nebulizer in a respiratory circuit without interrupting ventilation and for sealing the circuit when the nebulizer is removed. The apparatus of the invention incorporates structures and features which avoid the disadvantages of the device described in the aforesaid patent.

### Summary of the Invention

The present invention is directed to a valved aerosol Tee adapter assembly for removably securing a nebuliser in a Tee connector used in a respiratory circuit as recited in Claim 1. The device features a structure in which the valve spring is housed without exposure to or interference with the aerosol generated from the nebulizer. The structure also provides a substantially unobstructed channel for nebulized particles to pass into the Tee connector. The device includes a wall extending into the Tee connector passageway for preventing fluids and condensate from draining back into the nebulizer. These as well as other advantages and components of the assembly of the invention will be described in detail hereinafter.

### Brief Description of the Drawings

Fig. 1 is a sectional elevational view of the adapter assembly of the invention in a closed condition secured in a Tee connector;
Fig. 2 is a sectional elevation of the adapter assembly in an open condition in the Tee connector; and
Fig. 3 is a plan view of the lower end of the adapter assembly of the invention.

### Detailed Description of the Preferred Embodiments

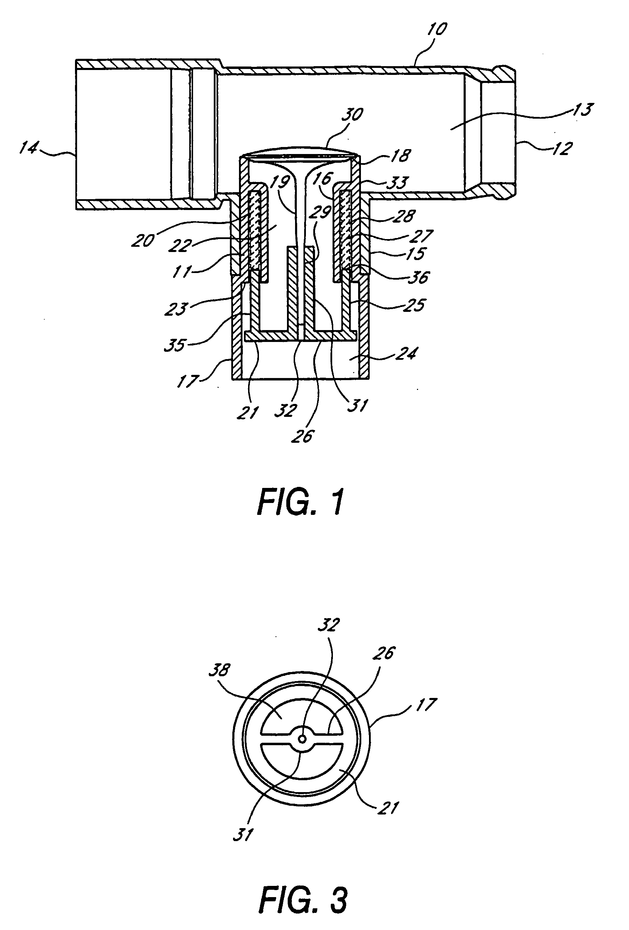

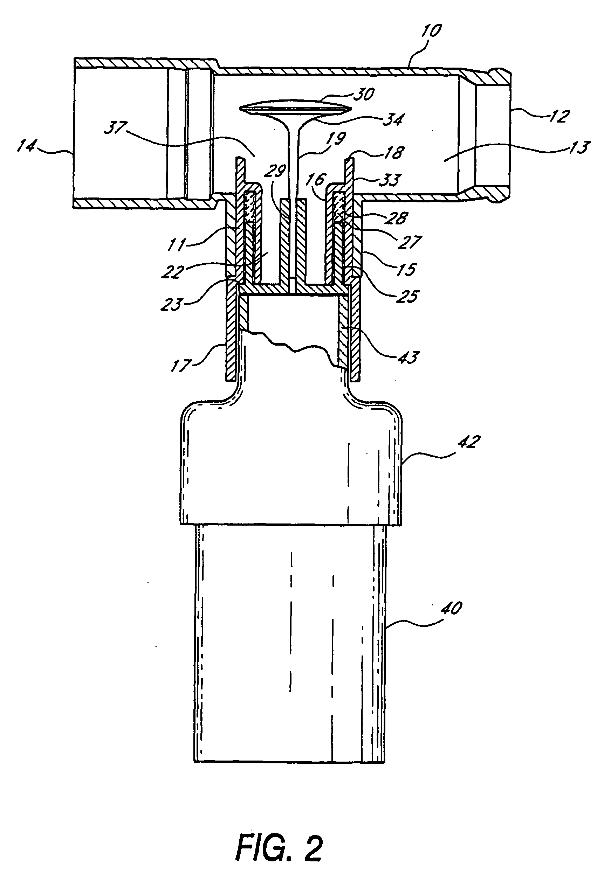

Figs. 1 and 2 illustrate the valved adapter assembly of the invention installed in a Tee connector 10. The Tee connector has inlet port 12 and outlet port 14 and interior passageway 13. The inlet and outlet ports are connected to ventilator breathing circuit hoses for supplying inspiratory gas from a ventilator or respirator to a patient. The valved assembly includes a housing having an upper section 11 and a lower section 17. In the preferred embodiment illustrated, the housing is substantially cylindrical with upper cylindrical section 11 having a smaller diameter than the cylindrical lower section 17. A cylindrical upper section allows the housing to be inserted with a gas sealing force-fit into the side port 15 of the Tee connector 10. A wall 16 is formed interiorly of the upper housing section 11. The space between wall 16 and inner wall surface 20 of upper section 11 forms the annular chamber 27 for housing a spring 28.

As shown in Fig. 1, valve 30 forms a gas seal against valve seat 18 along the upper edge of upper housing section 11. Valve actuator 25 comprises annular wall 35 extending from actuator ring 21. The upper edge 36 of annular wall 35 engages spring 28 which is biased in an expanded condition to close the valve. The valve actuator includes a valve support collar 31 into which a valve support member is positioned. In the embodiment illustrated, the valve support member includes a rod 19 integrally formed and extending downwardly from valve 30 and a lower rod portion 29 secured into channel 32 within collar 31. Preferably, the lower portion of rod 19 is tapered for a force-fit within channel 32. However, other features, shapes or components of the valve support member may be used for securing the valve to the valve actuator 25. The valve support collar 31 is integrally formed with the valve actuator and supported by ribs 26 extending between the collar and the valve actuator ring 21. By separately forming or molding the valve actuator and valve components, manufacture and assembly of the device is enhanced.

As shown in Fig. 2, a nebulizer 40 inserted in the lower section 17 of the housing urges the valve actuator and valve upperwardly from the closed position shown in Fig. 1 to the open position of Fig. 2. Fig. 2 illustrates the valve assembly of the invention in an open condition with valve actuator 25 moved upwardly in lower passageway 24 by urging aerosol outlet pipe 43 of nebulizer lid 42 into the passageway. The upper edge of outlet pipe 43 is forced against actuator ring 21 whereby continued upward pressure of the nebulizer forces the biased valve actuator to open the valve. The shoulder 23 forms a stop for upward movement of the valve actuator within lower passageway 24. With the valve actuator urged against shoulder 23 spring 28 is compressed and valve 30 is moved away from valve seat 18 so that upper passageway 22 within the upper section 11 of the valve housing communicates with the interior passageway 13 of the Tee connector 10. With the valve open, there is an unobstructed channel 37 between upper passageway 22 and the interior passageway 13 of the Tee connector for passage of aerosol from a nebulizer via aerosol outlet pipe 43 into the Tee connector. Aerosol from the nebulizer is directed through openings 38 in the valve actuator (see Fig. 3), along upper passageway 22 and unobstructed channel 37 into the Tee connector. The underside surface 34 of valve 30 is preferably arched or tapered to form a surface for better dispersing the aerosol uniformly within interior passageway 13 of the Tee connector. Preferably, the upper surface of the valve member is convex, as shown, to prevent pooling of condensate on the valve.

Preferably, the interior diameter of the lower passageway 24 provides a convenient force-fit with the aerosol outlet pipe of a nebulizer. This, lower passageway of the adapter assembly should be of a size so that the nebulizer may be easily attached and removed by hand. With the valved adapter assembly and Tee connector installed in a respiratory circuit, patient ventilation may be carried out normally without opening the circuit, or interrupting ventilation when a nebulizer is to be attached for administering aerosol treatment. Thereafter, the nebulizer is removed, again without interrupting patient ventilation. Examples of suitable nebulizers are shown and described in U.S. Patent Nos. 4,588,129 and Re. 33,642

An advantage of the device of the invention is that spring 28 does not obstruct the aerosol passageways, nor is it exposed to the aerosol thereby avoiding aerosol condensation on the spring and deterioration of the spring due to exposure to the aerosol. Another advantage of the device of the invention is that the upper section 11 of the housing extends into passageway 13 forming a barrier to prevent liquid from draining from the interior of the Tee connector into the nebulizer. Such a feature is shown with upper section wall 33 extending into the passageway 13 of the Tee connector 10 to form the liquid barrier. These and other advantages will be evident to those skilled in the art.

## Claims

1. A valved aerosol tee adapter assembly for removably securing a nebulizer in a Tee connector in a respiratory circuit, comprising:
a housing member having an upper section (11) having a first passageway (22) interiorly thereof and a lower section (17) having a second passageway (24) contiguous with said first passageway (22); and a valve seat (18) formed along the upper edge of said upper section (11), and
a valve reciprocally movable in said housing comprising a valve actuator (25) slidably received in said second passageway (24) having an upper surface (36) and a valve member (30) secured to said valve actuator (25), said assembly **characterized by**:
the upper section (11) including an inner wall (16) spaced apart from the inner wall surface (20) of said upper section (11) and defining an annular chamber (27) therebetween, and
a spring (28) positioned in said chamber (27), the upper surface (36) of said valve actuator (25) engaging said spring (28), the inner wall (16) and upper surface (36) together isolating the spring (28) from exposure to the first passageway (22).

2. An assembly of Claim 1 wherein said valve member (30) comprises a circular valve for sealingly engaging said valve seat (18) and one or more support members (19) extending from said valve and secured to said valve actuator (25).

3. An assembly of any preceding claim wherein said valve member (30) has a curved lower surface portion (34) exposed in said first passageway (22).

4. An assembly of Claim 3 including a single support member (19) extending from the center of the lower surface of said valve member (30) and secured to said valve actuator (25).

5. An assembly of Claim 3 including a plurality of support members extending from the lower surface of said valve member and secured to said actuator.

6. An assembly of Claim 4 wherein said single support member (19) is removably secured to said valve actuator (25).

7. An assembly of Claim 6 wherein said valve actuator (25) includes a collar (31) having a channel (32) therein for receiving said single support member (19).

8. An assembly of Claim 1, 2, 4, 5, 6 or 7 wherein said spring (28) is biased to urge said valve in sealing engagement with said valve seat.

9. An adapter assembly of Claim 8 and a Tee connector (10) having a side port and cylindrical fitting (15) communicating with an interior connector passageway (13) said adapter assembly secured into said fitting whereby part of the cylindrical upper section (11) of the housing extends into the connector passageway (13) to form a circular wall (33) therein.

10. An adapter assembly of Claim 8 and a Tee connector (10) having a side port and cylindrical fitting (15) communicating with an interior Tee connector passageway (13), said adapter assembly secured into said fitting whereby part of the cylindrical upper section (11) of the housing extends into the connector passageway (13) to form a circular wall (33) therein, and a nebuliser (40) secured in the lower section (17) of said adapter assembly against said valve actuator whereby said valve actuator (25) is moved in said second passageway (24) and said valve member (30) is spaced apart from said valve seat whereby said first passageway (22) is open to the interior Tee connector passageway (13).

## Patentansprüche

1. Mit einem Ventil versehene Aerosol-T-Adapter-Anordnung zur lösbaren Befestigung eines Zerstäubers in einem T-Verbindungsstück in einer Beatmungs-Einheit, mit:
einem Gehäuse-Teil, das einen oberen Abschnitt (11) mit einem ersten Kanal (22) in dessen Inneren und einen unteren Abschnitt (17) mit einem zweiten Kanal (24), der an den ersten Kanal (22) angrenzt; und einen Ventilsitz (18) aufweist, der entlang der Oberkante des oberen Abschnittes ausgebildet ist; und
einem Ventil, das in dem Gehäuse hin und her beweglich ist und ein Ventil-Betätigungsglied (25) umfasst, das in dem zweiten Kanal (24) verschiebbar aufgenommen ist und eine obere Oberfläche (36) und ein Ventil-Element (30) aufweist, das an dem Ventil-Betätigungsglied (25) befestigt ist, wobei die Anordnung **dadurch gekennzeichnet ist, dass**:
der obere Abschnitt (11) eine Innenwand (16) einschließt, die mit Abstand von der inneren Wandoberfläche (20) des oberen Abschnittes (11) angeordnet ist und eine ringförmige Kammer (27) zwischen diesen Teilen begrenzt; und
eine Feder (28), die in der Kammer (27) angeordnet ist, wobei die obere Oberfläche (36) des Ventil-Betätigungsgliedes (25) mit der Feder (28) in Eingriff steht und die Innenwand (16) und die obere Oberfläche (36) zusammen die Feder (28) gegenüber einer Beaufschlagung durch den ersten Kanal (22) isolieren.

2. Anordnung nach Anspruch 1, bei der das Ventil-Element (30) ein kreisförmiges Ventil für einen Dichtungseingriff mit dem Ventilsitz (18) und ein oder mehrere Halterungsteile (19) umfasst, die sich von dem Ventil aus erstrecken und an dem Ventil-Betätigungsglied (25) befestigt sind.

3. Anordnung nach einem der vorhergehenden Ansprüche, bei dem das Ventil-Element (30) einen gekrümmten unteren Oberflächenabschnitt (34) aufweist, der in dem ersten Kanal (22) freiliegt.

4. Anordnung nach Anspruch 3, die ein einziges Halterungsteil (19) einschließt, das sich von dem Mittelpunkt der unteren Oberfläche des Ventil-Elementes (30) aus erstreckt und an dem Ventil-Betätigungsglied (25) befestigt ist.

5. Anordnung nach Anspruch 3, die eine Vielzahl von Halterungsteilen einschließt, die sich von der unteren Oberfläche des Ventil-Elementes aus erstrecken und an dem Betätigungsglied befestigt sind.

6. Anordnung nach Anspruch 4, bei der das einzige Halterungsteil (19) lösbar an dem Ventil-Betätigungsglied (25) befestigt ist.

7. Anordnung nach Anspruch 6, bei der das Ventil-Betätigungsglied (25) einen Bundring (31) mit einem darin angeordneten Kanal (32) zur Aufnahme des einzigen Halterungsteils (19) einschließt.

8. Anordnung nach einem der Ansprüche 1, 2, 4, 5, 6 oder 7, bei der die Feder (28) vorgespannt ist, um das Ventil in Dichtungseingriff mit dem Ventilsitz zu drücken.

9. Eine Adapter-Anordnung nach Anspruch 8 und ein T-Verbindungsstück (10) mit einem seitlichen Anschluss und einem zylindrischen Passstück (15), die mit einem innenliegenden Verbindungsstück-Kanal (13) in Verbindung stehen, wobei die Adapter-Anordnung in dem Passstück befestigt ist, wodurch ein Teil des zylindrischen oberen Abschnittes (11) des Gehäuses sich in den Verbindungsstück-Kanal (13) erstreckt, um in diesem eine kreisförmige Wand (33) zu bilden.

10. Eine Adapter-Anordnung nach Anspruch 8 und ein T-Verbindungsstück (10) mit einem seitlichen Anschluss und einem zylindrischen Anschlussstück (15), das mit einem innenliegenden T-Verbindungsstück-Kanal (13) in Verbindung steht, wobei die Adapter-Anordnung in dem Anschlussstück befestigt ist, wodurch ein Teil des zylindrischen oberen Abschnittes (11) des Gehäuses sich in dem Verbindungsstück-Kanal (13) erstreckt, um eine kreisförmige Wand (33) hierin zu bilden, wobei ein Zerstäuber (40) in dem unteren Abschnitt (17) der Adapter-Anordnung gegen das Ventil-Betätigungsglied befestigt ist, wodurch das Ventil-Betätigungsglied (25) in dem zweiten Kanal (24) bewegt wird und das Ventil-Element (30) mit Abstand von dem Ventilsitz angeordnet wird, wodurch der erste Kanal (22) zu dem innenliegenden T-Verbindungsstück-Kanal (13) hin offen ist.

## Revendications

1. Ensemble d'adaptateur en forme de T pour aérosol à valve pour fixer de manière amovible un pulvérisateur dans un connecteur en forme de T dans un circuit respiratoire, comprenant :
un élément de boîtier ayant une section supérieure (11) ayant une première voie de passage (22) à l'intérieur de celle-ci et une section inférieure (17) ayant une seconde voie de passage (24) contiguë à ladite première voie de passage (22) ; et un siège (18) de valve formé le long du bord supérieur de ladite section supérieure (11), et
une valve mobile selon un mouvement de va-et-vient dans ledit boîtier comprenant un actionneur (25) de valve reçu de manière coulissante dans ladite seconde voie de passage (24) ayant une surface supérieure (36) et un élément de valve (30) fixé sur ledit actionneur (25) de valve, ledit ensemble étant **caractérisé par** :
la section supérieure (11) comprenant une paroi interne (16) espacée de la surface de paroi interne (20) de ladite section supérieure (11) et définissant une chambre annulaire (27) entre elles, et
un ressort (28) positionné dans ladite chambre (27), la surface supérieure (36) dudit actionneur (25) de valve engageant ledit ressort (28), la paroi interne (16) et la surface supérieure (36) isolant ensemble le ressort (28) de l'exposition à la première voie de passage (22).

2. Ensemble selon la revendication 1, dans lequel ledit élément de valve (30) comprend une valve circulaire pour engager de manière étanche ledit siège (18) de valve et un ou plusieurs éléments de support (19) s'étendant à partir de ladite valve et fixés audit actionneur (25) de valve.

3. Ensemble selon l'une quelconque des revendications précédentes, dans lequel ledit élément de valve (30) a une partie de surface inférieure incurvée (34) exposée dans ladite première voie de passage (22).

4. Ensemble selon la revendication 3, comprenant un élément de support unique (19) s'étendant à partir du centre de la surface inférieure dudit élément de valve (30) et fixé audit actionneur (25) de valve.

5. Ensemble selon la revendication 3, comprenant une pluralité d'éléments de support s'étendant à partir de la surface inférieure dudit élément de valve et fixés audit actionneur.

6. Ensemble selon la revendication 4, dans lequel ledit élément de support unique (19) est fixé de manière amovible audit actionneur (25) de valve.

7. Ensemble selon la revendication 6, dans lequel ledit actionneur (25) de valve comprend un collier (31) ayant un canal (32) à l'intérieur de celui-ci pour recevoir ledit élément de support unique (19).

8. Ensemble selon la revendication 1, 2, 4, 5, 6 ou 7, dans lequel ledit ressort (28) est sollicité pour pousser ladite valve dans un engagement d'étanchéité avec ledit siège de valve.

9. Ensemble d'adaptateur selon la revendication 8 et connecteur en forme de T (10) ayant un orifice latéral et un raccord cylindrique (15) communiquant avec une voie de passage de connecteur interne (13), ledit ensemble d'adaptateur étant fixé dans ledit raccord, moyennant quoi une partie de la section supérieure cylindrique (11) du boîtier s'étend dans la voie de passage (13) de connecteur pour former une paroi circulaire (33) à l'intérieur de celle-ci.

10. Ensemble d'adaptateur selon la revendication 8 et connecteur en forme de T (10) ayant un orifice latéral et un raccord cylindrique (15) communiquant avec une voie de passage de connecteur en forme de T interne (13), ledit ensemble d'adaptateur étant fixé dans ledit raccord, moyennant quoi une partie de la section supérieure cylindrique (11) du boîtier s'étend dans la voie de passage de connecteur (13) pour former une paroi circulaire (33) à l'intérieur de celte-ci, et un pulvérisateur (40) fixé dans la section inférieure (17) dudit ensemble d'adaptateur contre ledit actionneur de valve, moyennant quoi ledit actionneur de valve (25) est déplacé dans ladite seconde voie de passage (24) et ledit élément de valve (30) est espacé dudit siège de valve moyennant quoi ladite première voie de passage (22) est ouverte sur la voie de passage de connecteur en forme de T interne (13).
